# EUROPEAN PATENT APPLICATION

(11) **EP 2 982 733 A1**
(43) Date of publication of application: **10.02.2016**
(21) Application number: 14179904.9
(22) Date of filing: 05.08.2014
(51) Int. Cl.: C10G 1/00, C11B 1/02, C11B 1/10, C10G 1/02, C10G 21/16, C10G 21/14

(54) **Method of recovering oleagineous compounds from hydrothermally biomass**

(71) Applicant: Linde Aktiengesellschaft, 80331 München (DE)
(72) Inventor: Kistenmacher, Hans, 82152 Planegg (DE); Alba, Laura Garcia, 81639 München (DE); Lauermann, Gerhard, 81379 München (DE); Simon, Johann-Guenter, 81671 München (DE); Aman, Lidia, 01237 Dresden (DE); Kleeberg, Joerg, Dr., 09600 Oberschöna OT Kleinschirma (DE)
(74) Representative: m patent group

(57) **Abstract**

A method of recovering oleagineous compounds from hydrothermally treated biomass, including providing a biomass feedstock as an aqueous biomass suspension and processing the biomass suspension at a reaction temperature and a reaction pressure for a reaction time in a reactor to produce a product mixture is provided. In a separation step, the product mixture is contacted with an extraction fluid comprising at least one extraction agent selected from the group consisting of monoaromatic hydrocarbons, monoaromatic alcohols and monoaromatic ethers to obtain an organic liquid phase that contains at least a part of the oleagineous compounds. Use of at least one corresponding extraction agent in a method of recovering oleagineous compounds from hydrothermally treated biomass is also subject of the invention.

## Description

The invention relates to a method of recovering oleagineous compounds from hydrothermally treated biomass and to the use of specific extraction agents in such a method.

### Prior art

Aquatic algae are capable of producing much larger amounts of biomass than terrestrial plants on the same area but do not require freshwater and/or arable land. Especially algae producing oleagineous compounds are an attractive source of renewable fuels. From such algae, oleagineous compounds that are already present in the cells can be extracted. Furthermore, the partial conversion of the whole algal biomass into oleagineous compounds is also possible.

After harvesting, algae are dried and their oleagineous compounds are extracted using conventional vegetable oil extraction processes with suitable organic solvents such as hexane (see R.W. Ozer, "Algae Drying and Extraction", in: W. Farr and A. Procter, Eds.: "Green Vegetable Oil Processing", Urbana 2012, AOCS Press).

Alternatively, wet algal slurries are processed directly in the wet phase at high temperatures by hydrothermal liquefaction (HTL, see D.C. Elliott et al., "Process Development for Hydrothermal Liquefaction of Algae Feedstocks in a Continuous-Flow Reactor", Algal Res. 2, 2013, 445-454 and D.C. Elliott et al., "Hydrothermal Processing of Macroalgal Feedstocks in Continuous-Flow Reactors"; ACS Sustainable Chem. Eng. 2, 2014, 207-215). HTL processes involve the reaction of biomass with high temperature water at high pressure for a defined reaction time in presence or absence of a catalyst. The product mixture thus obtained contains water, oleagineous compounds and solids. A fraction of the oleagineous compounds obtained is soluble in water and the other fraction may form a separate liquid phase.

HTL is generally regarded as superior over dry extraction because it is not necessary to spend a large amount of energy to remove the water which amounts to 80 to 95% of the algal slurry. In addition, the mixture of oleagineous compounds in the HTL product mixture is not only produced by the oleagineous compounds present as such in the algae but also converted from other algae components containing hydrocarbon fragments like cell walls, proteins, etc.

Conventional methods, relying mostly on gravity separation of water rich and oleagineous compounds rich liquid phases, do not allow for a complete recovery of the oleagineous compounds from the HTL product mixture. A considerable amount of valuable oleagineous components is thus left in the water phase and lost to successive processes. In addition, emulsions are formed between the oleagineous and the water rich phases unless appropriate measures are taken such as the removal of solids or the addition of emulsion breakers. Known solvents used in dry extraction methods are also not satisfactory either (see below).

It is therefore an object of the present invention to provide a more effective method of recovering oleagineous compounds from hydrothermally treated biomass.

### Summary of the invention

According to the invention, a method of recovering oleagineous compounds from the HTL product mixture and the use of specific extraction agents in such a method, as defined in the independent claims, is suggested. Preferred embodiments of the invention are subject of the dependent claims and of the description that follows.

The present invention is explicitly not limited to algal suspensions but can also be used for other types of biomass, e.g. sludge, manure, food or feedstock byproducts and mixtures thereof. The invention is, however, particularly useful for recovering oleagineous compounds from hydrothermally treated pro- or eukaryotic, especially aquatic, organisms.

Examples fur such organisms are red, green or brown macroalgae or microalgae and bacteria. Macroalgae and Microalgae usable in the present invention can include species (sp.) from the genera *Desmodesmus, Chlamydomonas, Dunaliella, Haematococcus, Scenedesmus, Chlorella* or *Nannochloropsis.* Bacteria can include species from the genera *Synechococcus, Synechocystis, Athrospira, Prochlorococcus, Chroococcus, Gleoecapsa, Aphunocapsu, Aphanothece, Leptolyngbya, Merismopedia, Microcystis, Coelosphaeritan, Prochlorothrix, Oscillatoria, Trichodesmium, Spirulina, Microcoleus, Chroococcidiopisis, Anabaena, Aphanizamenon, Cylindindrospermopsis, Cylindrospermum, Tolypothrix* or *Scytonema.*

In hydrothermal liquefaction, compounds derived from the lipid fraction of the biomass, e.g. of an algal suspension, but also a comparatively large amount of compounds derived from other precursor fractions, e.g. from proteins and carbohydrates, are obtained. These compounds are, however, only in part obtained in an unchanged form, i.e. were present as such in the biomass, but to a large extent underwent substantial thermal decomposition (e.g. fatty acids break into hydrocarbons, etc.).

The so-called "oil" obtained in hydrothermal liquefaction is thus a complex mixture of compounds which mainly are derived from the lipid (e.g. hydrocarbons), the protein (e.g. nitrogen containing components) and the carbohydrate fractions (e.g. sugars) of the processed biomass, e.g. of algal cells. The term "oleaginous compounds" shall therefore, in the present application, refer to such compounds, which typically have hydrophobic characteristics.

### Advantages of the invention

The invention is based on the surprising finding that specific solvents (in the following referred to as "extraction agents"; both terms are used synonymously) are especially suitable for extracting oleagineous compounds from HTL product mixtures at low solvent to water ratios, thus increasing the oleagineous compounds recovery while requiring only a small amount of solvent.

As mentioned above, conventional methods for recovering oleagineous compounds from HTL product mixtures, relying mostly on the gravity separation of water rich and oleagineous compounds rich phases, do not allow for a complete recovery of the oleagineous compounds. Experimental work showed that, even if the solids were removed in advance, in such methods only about 30% of the biomass could be recovered as oleagineous compounds and more than 10% of the biomass that was converted into oleagineous compounds was left in the product water phase. This large amount of oleagineous compounds lost makes the HTL process nearly uneconomical, despite its general advantages.

The present invention, in contrast, providing significantly higher extraction yields, combines the advantages of the HTL process, namely that there is no need to evaporate large amounts of water, with the advantages of a solvent extraction process, which allows recovering nearly all oleagineous compounds that are present in the HTL product mixture.

In US 8,192,628 B2 it is proposed to use hexane (like typically in dry oil extraction) as an extraction solvent. However, the amount of hexane needed to recover about 90% of the oleagineous compounds contained in the HTL product mixture is more than five times the amount of the HTL product mixture. As, in order to recover the oleagineous compounds from this large amount of solvent, the solvent needs to be evaporated, more energy is required for evaporation than is contained in the recovered oleagineous compounds. Therefore, the hexane extraction is not feasible based on the large amount of fossil fuel needed to recover more than 90% of the renewable oleagineous compound mixture. Dichloromethane (DCM) was also suggested as an extraction solvent. However, in refinery processing, the presence of DCM is disadvantageous because of the danger of irreversible damage to the refinery construction material, being generally sensitive to any chlorine content. Additionally, chlorinated compounds are generally considered hazardous.

The specific extraction agents proposed according to the present invention, in contrast, avoid these disadvantages and still allow recovering in an economical way the overwhelming part of the olagineous compounds that have been produced by the conversion of the biomass, especially in a HTL process.

To arrive at the present invention, with a sophisticated and exhaustive combination of theoretical and selective experimental work, more than 1 000 possible solvents (extraction agents) were studied in different levels of detail. Based on these results, monoaromatic hydrocarbons, monoaromatic alcohols and monoaromatic ethers were found to perform better than the rest. While, in the following, results are shown for the representative compounds toluene, benzyl alcohol, methoxybenzene, ethoxybenzene, o-xylene, m-xylene, p-xylene and mesitylene, it is to be noted that monoaromatic hydrocarbons, monoaromatic alcohols and monoaromatic ethers are generally suitable for use in the present invention. Under "monoaromatic hydrocarbon", a compound comprising exactly one aromatic ring and consisting only of hydrogen and carbon atoms is to be understood. Correspondingly, "monoaromatic alcohols" and "monoaromatic ethers" are alcohols or ethers comprising exactly one aromatic ring.

The work used a solvent screening model which is based on an experimentally identified organic composition of a HTL product mixture generated from a *Desmodesmus* sp. algal culture (see L. Garcia Alba, "Algae Biorefinery, an Experimental Study on Liquid Fuels Production and Nutrients Recycling", Dissertation, University of Twente, 2013). This composition is regarded as typical for a HTL liquid mixture. However, as mentioned, the results underlying the present invention are considered also applicable to other organisms showing comparable compositions of olagineous compounds.

Several thermodynamic methods were used and combined to predict the physical properties for the extraction process step. The model used for the phase equilibria calculations was a cubic equation of state for the gas phase and a modified universal quasichemical (UNIQUAC) model for the prediction of the activity coefficients in the liquid phases (solvent and water). In general, applicable experimental activity coefficient data were preferentially used to fit the models to the data. If reliable parameters from the modified universal quasichemical functional group activity coefficient (UNIFAC) matrix were available, these parameters were used to generate the UNIQUAC parameters. Finally, when neither experimental data nor reliable UNIFAC parameters were available, activity coefficients were calculated by the COSMOtherm software and the remaining UNIQUAC parameters were determined in this way. A wide range of extraction temperatures (from 25 up to 100 °C) and solvent to water mass ratios (from 0.004:1 to 6:1) were evaluated.

After performing the simulations, the candidate solvents (extraction agents) and the corresponding extraction results were evaluated via several criteria. Briefly, significantly lower recovery of oleagineous compounds than with the standard solvent DCM, toxicity, legal and/or environmental problems involved with the candidate solvents, and/or large amounts of the candidate solvents being lost into the water phase and vice versa led to exclusion from further consideration. For the remaining solvent candidates, studies regarding the optimum extraction conditions were performed. The results are given in the figures and discussed below.

Based on the findings of the process as explained above, the current invention suggests an improved method of recovering oleagineous compounds from hydrothermally treated biomass with the use of specific extraction agents.

According to the invention, the product mixture is, in a separate step, contacted with an extraction fluid comprising at least one specific solvent (extraction agent) to obtain an organic liquid phase containing the oleagineaous compounds. As mentioned before, the present invention shows that especially monoaromatic hydrocarbons, monoaromatic alcohols and monoaromatic ethers, especially toluene, benzyl alcohol, methoxybenzene, ethoxybenzene, o-xylene, m-xylene, p-xylene and mesitylene are especially suitable as extraction agents for the extraction of oleagineous compounds from hydrothermally treated biomass. According to the present invention, in a separation step the product mixture is therefore contacted with an extraction fluid comprising at least one extraction agent selected from the group consisting of monoaromatic hydrocarbons, monoaromatic alcohols and monoaromatic ethers to obtain an organic liquid phase that contains at least a part of the oleagineous compounds. The specific choice between the extraction agents mentioned above will largely depend on the downstream process configuration and e.g. the composition of the oleagineous compounds mixture targeted. For instance, toluene will, as will be explained below, require less energy for evaporation, but with the penalty of recovering less oleagineous compounds out of the HTL product mixture. In contrast, benzyl alcohol can achieve almost 100% recovery of oleagineous compounds but also has a very high boiling point requiring a large amount of energy for its total evaporation. However, rather of being a disadvantage, this high boiling point could be used as a tool for the fractionation of the oleagineous compounds between light and heavier fractions by e.g. distilling the light oleaginous compounds together with the benzyl alcohol.

As mentioned above, the invention is not limited to the specific compounds mentioned above. Rather, a group of compounds showing comparable chemical characteristics is considered as being suitable for extraction according to the present invention. Generally, the at least one extraction agent used according to the present invention may advantageously have a molar mass between 92 and 160 g/mol.

An extraction fluid used according to the present invention may comprise at least 75, 80 or 95 vol% of the specific extraction agent. The extraction agents may also be used as substantially pure substances, with the typical amounts of byproducts or contaminants of technical or analytical solvents.

Especially in comparison with the use of hexane which has, as mentioned above, to be used in comparatively large amounts in relation to the product mixture from which the oleagineous compounds are to be extracted, the extraction agents mentioned may be used in a much lower amount in relation to the product mixture. For example, the extraction fluid can be used in an amount from 0.004:1 to 6:1 of solvent to water ratio, especially in an amount from 0.1:1 to 1:1 of solvent to water ratio. The exact amounts to be used depend on the intended extraction yield, which can, as explained below, increase to 90% or more with a significantly lower solvent amount than that required for hexane. For instance, only a solvent to water ratio of 0.05:1 is required with benzyl alcohol to achieve an oleagineous compounds recovery of 90% while, for hexane, a 5:1 ratio is needed to achieve that same yield.

According to an especially preferred embodiment of the invention, the separation step includes agitating the product mixture and the extraction fluid after contacting them with the extraction fluid with a specifc extraction time, i.e. by stirring, shaking and the like.

It can also be advantageous to contact the product mixture and the extraction fluid in a fractionating and/or extraction device in the separation step. Suitable fractionation and/or extraction devices are known to the skilled person and may e.g. include phase exchange features like trays, sieves, spraying devices and the like. By using corresponding devices, the extraction speed may be increased.

As generally known, after this separation step the organic solvent phase may be at least partially evaporated to recover the oleagineous compounds. As mentioned, depending on the degree of evaporation and/or the downstream processing steps, one of the extraction agents might be favoured as extraction fluids.

As mentioned, the invention is especially useful for hydrothermally treated biomass, i.e. processes involving e.g. reaction temperatures of 175 to 450 °C, reaction pressures from 10 to 30 MPa and/or reaction times of 2 to 60 minutes. Such processes can especially be realized with continuous flow reactors as disclosed in the literature mentioned above.

The current invention also relates to the use of at least one extraction agent in a method of recovering oleagineous compounds from hydrothermally treated biomass that is provided as aqueous biomass suspension and processed at a reaction temperature and a reaction pressure for a reaction time in a reactor to produce a product mixture. According to the present invention, the at least one extraction agent is selected from the group consisting of monoaromatic hydrocarbons, monoaromatic alcohols, monoaromatic ethers and mixtures thereof and the at least one extraction agent is used in an extraction fluid which is contacted with the product mixture in a separation step to obtain an organic liquid phase that contains at least a part of the oleagineous compounds.

Preferentially, the at least one extraction agent is selected from the group consisting of toluene, benzyl alcohol, methoxybenzene, ethoxybenzene, m-xylene, o-xylene, p-xylene and mesitylene. For specifics and specific advantages of the inventive use, it is explicitly refered to the explanations above.

The invention and preferred embodiments of the invention will be described with reference to the appended figures, showing details of the current invention.

### Short description of the figures

Figure 1 shows a criteria scheme for solvent selection according to a preferred embodiment of the invention.
Figure 2 shows a diagram indicating the mass percentage of oleagineous compounds recovered by extraction with different extraction agents by increasing the solvent to water ratio.
Figures 3A and 3B show water dissolved in a solvent phase and vice versa after extraction with different extraction agents at increasing solvent to water ratio.
Figure 4 shows a diagram indicating the energy required to evaporate a extraction agent according to a preferred embodiment of the invention.

### Embodiments of the invention

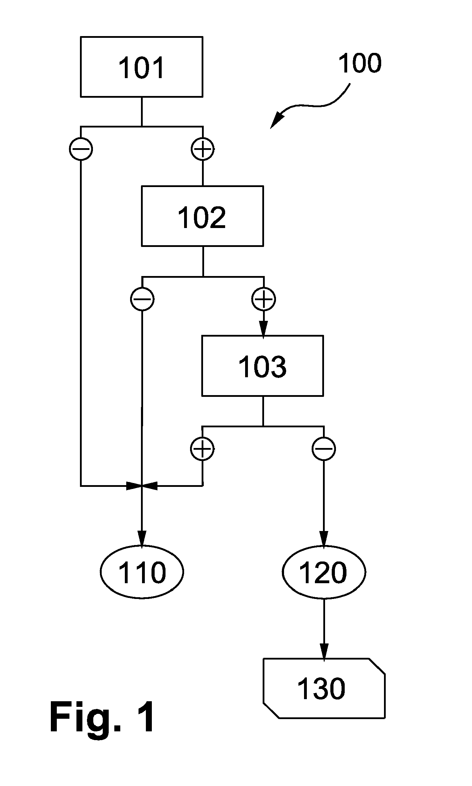

In Figure 1, a criteria scheme to find an optimum solvent or optimum solvents for use in extraction of oleagineous compounds from HTL-product mixtures is displayed and generally indicated with 100.

In a step 101 of the scheme 100, which was performed after, as mentioned, thermodynamic modelling steps have already been performed by using UNIFAC, UNIQUAC and COSMOtherm simulation methods, it was examined whether the candidate solvent (extraction agent) shows a similar or higher recovery of oleagineous compounds, here oleagineous compounds from *Desmodesmus* sp. as mentioned above, as the standard solvent DCM. DCM has been widely used in the literature as it has shown to recover large amounts of oleagineous compounds when compared to solvent free separation. Consequently, in step 101 all solvents having a recovery performance significantly lower than DCM (-) were excluded as indicated with decision block 110. Solvents showing a higher or similar extraction efficiency as compared to DCM (+) were examined in a step 102.

In Step 102, legislative regulations on the usage of candidate solvents and possible environmental hazards were evaluated. In step 102, candidate solvents that are toxic and/or dissallowed and/or that could cause large environmental damage (-) were excluded. It is to be noted that e.g. DCM was only used as a standard but never chosen as a potential extraction agent because chlorinated compounds are known to be toxic and hazardous substances. For similar reasons, methyl isobutyl ketone (MIBK) was excluded.

Subsequently, for the remaining candidates, in a step 103 the amount of solvent that dissolves into water and vice versa for the candidates left were evaluated. High economic implications could be caused by losing a large amount of solvent due to the high energy required to drive it off the water. Similar, the water has to be purified to be able to recycle it, e.g. into an algae cultivation unit. Consequently, solvents exhibiting a large solubility in water, as mentioned above, were excluded (+). Solvents with a low solubility in water and vice versa (-) were, in a decision step 120, taken as optimum solvents for the task mentioned. These solvents are, as mentioned, monoaromatic hydrocarbons, monoaromatic alcohols and/or monoaromatic ethers such us toluene, benzyl alcohol, methoxybenzene, ethoxybenzene, o-xylene, m-xylene, p-xylene and mesitylene. In a further step 130, extraction conditions were evaluated as in detail explained below.

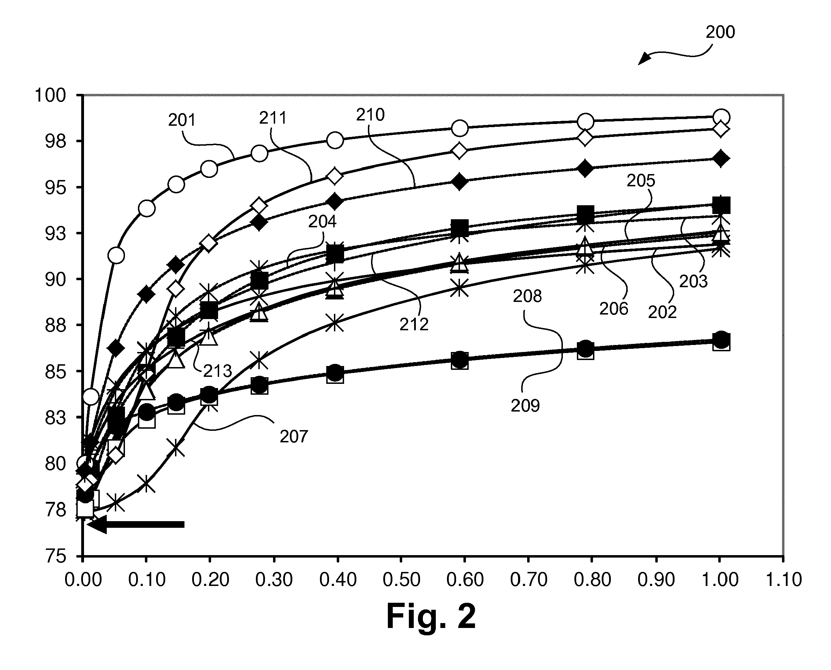

In Figure 2, the recovery of the sum of all oleagineous compounds in the solvent phase from the HTL product mixture after extraction with different solvents are given in wt% on the ordinate versus increasing solvent to water ratios on the abscissa in a diagram 200. The composition of the HTL product mixture used as input was close to that of the product mixture from HTL of *Desmodesmus sp.* (shown in L. Garcia Alba, "Algae Biorefinery, an Experimental Study on Liquid Fuels Production and Nutrients Recycling", Dissertation, University of Twente, 2013).

Results are shown for benzyl alcohol 201 (empty circles), toluene 202 (diagonal crosses), DCM 203 (asterisks, dotted line), MIBK 204 (filled squares), propyl acetate 205 (empty triangles), isopropyl acetate 206 (filled triangles), ethyl acetate 207 (asterisks, solid line), hexane 208 (filled circles), cyclohexane 209 (empty squares), methoxybenzene (210, filled diamonds), benzylamine 211 (empty diamonds), m-xylene 212 (regular crosses, dotted line) and mesitylene 213 (regular crosses, solid line). Heptane, decane and hexadecane performed almost equal to hexane, chloroform performed almost equal to DCM, o-xylene and p-xylene performed almost equal to m-xylene and ethoxybenzene performed almost equal to methoxybenzene, therefore, results for these solvents are not shown in Figure 2. An arrow indicates the extraction yield without solvent. Only the results from a 100 °C extraction temperature are presented because all solvents showed to perform better at high temperature. However, depending on the downstream configuration, lower temperatures might be preferable (e.g. for a better heat integration). Yet the trends shown in Figure 2 would remain the same.

As can be seen from Figure 2, solvents such as ethyl acetate 207, cyclohexane 209 and hexane 208 show significantly lower oleagineous compounds recovery compared to that of DCM 203. These solvents were not further investigated. In addition, for some of the solvents, the oleagineous compounds recovery curve had a flatter region at low solvent to water ratios (from 0.004:1 to about 0.05:1). This can be seen in Figure 2 particularly for ethyl acetate 207. This flatter region of the curve is caused by the fact that, if a small amount of solvent is added, most of it immediately dissolves into the large amount of water and therefore no solvent is left for extraction. The following table shows the example for ethyl acetate 207. At a solvent to water ratio of 0.05:1, 92 % of the solvent dissolves into the water and therefore only 12.6 kg/h of solvent is available to extract the 100 kg/h of oleagineous compounds:

| Solvent to water ratio | Solvent added for extraction (kg/h) | Solvent that physically dissolves into the water (kg/h) | Solvent left for extraction (kg/h) |
|---|---|---|---|
| 0.004:1 | 10 | 9.22 | 0.78 |
| 0.01:1 | 30 | 27.60 | 2.40 |
| 0.05:1 | 130 | 117.39 | 12.61 |

In contrast, e.g. benzyl alcohol 201 revealed an outstanding performance achieving almost 98 wt% oleagineous compounds recovery at comparatively low solvent to water ratios. The rest of the solvents exhibit superior or similar performances to DCM 203 and, therefore, were not yet excluded and went through the next step (cf. step 102 in Figure 1) of the criteria scheme applied for solvent optimization.

As mentioned, legal regulations on the use of the selected solvents were evaluated and toxic substances were excluded from further studies. Only unproblematic substances were further evaluated and tested for water solubility (cf. step 103 in Figure 1).

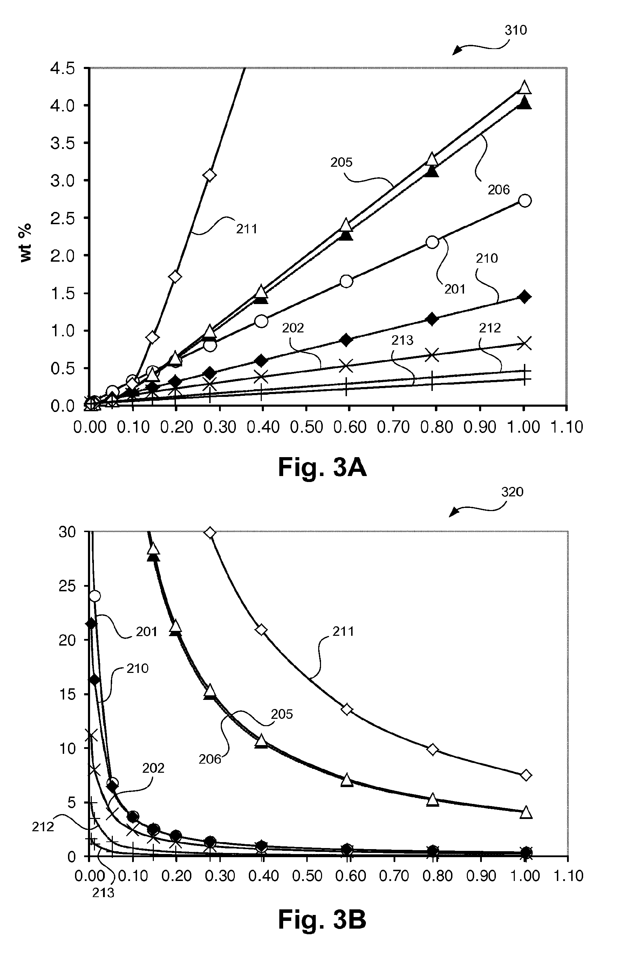

Figures 3A and 3B show water dissolved in the solvent phase (Figure 3A, diagram 310) and solvent dissolved into the water phase (Figure 3B, diagram 320) in wt% on the ordninates versus increasing solvent to water ratios on the abscissas. The solvents are indicated with the same symbols and reference numerals as in Figure 2. Benzyl alcohol 201 (empty circles), toluene 202 (diagonal crosses), propyl acetate 205 (empty triangles), isopropyl acetate 206 (filled triangles), methoxybenzene (210, filled diamonds), benzylamine 211 (empty diamonds), m-xylene 212 (regular crosses, dotted line) and mesitylene 213 (regular crosses, solid line) are indicated, these solvents representing the solvents still included in the study after step 102 (cf. Figure 1). As mentioned above, o-xylene and p-xylene performed almost equal to m-xylene and ethoxybenzene performed almost equal to methoxybenzene and no results for these solvents are shown.

The results displayed in Figures 3A and 3B clearly show that propyl aceteate 205, isopropyl acetate 206 and benzylamine must be excluded. Especially when looking at Figure 3B, showing high amounts of propyl aceteate 205, isopropyl acetate 206 and benzylamine 211 lost in the water phase at lower solvent to water ratios, it becomes evident that a relatively high amount of these solvents will be lost during extraction. benzylamine 211 was the solvent with best performed in terms of oleagineous compounds recovery after benzyl alcohol 201. Unfortunately, its solubility in water is too high.

Consequently, the best solvents for the presently studied case are monoaromatic hydrocarbons, monoaromatic alcohols and/or monoaromatic ethers such as toluene, benzyl Alcohol, methoxybenzene, ethoxybenzene, o-xylene, m-xylene, p-xylene and mesitylene. In other words, these solvents identified will lead to an environmentally and economically feasible HTL-process. Especially, benzyl alcohol 201 has shown a dramtically higher oleagineous compounds recovery when compared to the rest of the solvents, achieving almost 100% recovery. To the best of our knowledge, these solvents have never been proposed in the scientific literature or any other public report for the purpose described here.

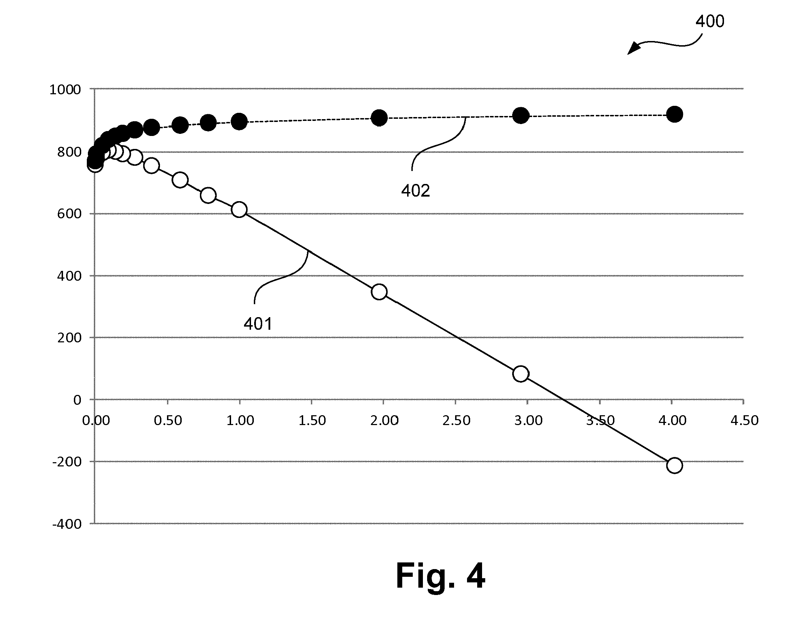

Regarding the extraction conditions, higher temperatures (above 100 °C) were investigated for both solvents, but no further increase in oleagineous compounds recovery was observed. The optimum solvent to water ratio will depend on the established target for parasitic load, i.e. the maximum amount of energy that can be consumed by the process as compared to the net energy production. The net energy production is the amount of energy left after substracting the energy required mainly to evaporate the solvent (main cost driver) from the energy content of the produced oleagineous compounds mixture. An example is shown in Figure 4 (in terms of power) for extraction with toluene at 100°C. In diagram 400 of Figure 4, again the solvent to water ratio is indicated on the abscissa and the energy consumption is indicated in kW on the ordinate. For simplicity, only the results for toluene are shown. Results are displayed for net energy consumption 401 and energy content in the oleagineous compounds mixture 402. As can be seen from Figure 4, above a solvent to water ratio of about three, more energy is required to evaporate the solvent than the energy content of the oleagineous compounds mixture produced.

The choice between the various solvents will depend on the downstream process configuration wanted and the composition of the oleagineous compounds mixture targeted. For instance, toluene 202 will require less energy for evaporation but with the penalty of recovering less oleagineous compounds out of the HTL product mixture. In contrast, benzyl alcohol 201 can achieve almost 100% oleagineous compounds recovery but has also a rather high boiling point requirement with large amounts of energy for its total evaporation. However, rather of being a disadvantage, this high boiling point could be used as a tool for the fractionation of the oleagineous compounds between e.g. light and heavy fractions.

## Claims

1. A method of recovering oleagineous compounds from hydrothermally treated biomass, including providing a biomass feedstock as an aqueous biomass suspension and processing the biomass suspension at a reaction temperature and a reaction pressure for a reaction time in a reactor to produce a product mixture, **characterized in that** in a separation step the product mixture is contacted with an extraction fluid comprising at least one extraction agent selected from the group consisting of monoaromatic hydrocarbons, monoaromatic alcohols and monoaromatic ethers to obtain an organic liquid phase that contains at least a part of the oleagineous compounds.

2. A method according to claim 1, wherein the at least one extraction agent is selected from the group consisting of toluene, benzyl alcohol, methoxybenzene, ethoxybenzene, m-xylene, o-xylene, p-xylene and mesitylene.

3. A method according to claim 1 or 2, wherein the at least one extraction agent has a molar mass between 92 and 160 g/mol.

4. A method according to any one of the preceding claims, wherein the extraction fluid comprises at least 75, 80, 90 or 95 vol% of the at least one extraction agent.

5. A method according to any one of the preceding claims, wherein in the separation step an amount of the product mixture and an amount of the extraction fluid is used, wherein the amount of the extraction fluid is from 0.004:1 to 6:1 of solvent to water ratio, especially in an amount from 0.1:1 to 1:1 of solvent to water ratio.

6. A method according to any one of the preceding claims, wherein the separation step includes agitating the product mixture and the extraction fluid after contacting for an extraction time.

7. A method according to any one of the preceding claims, wherein the separation step includes contacting the product mixture and the extraction fluid in a fractional extraction device.

8. A method according to any one of the preceding claims, wherein the separation step includes heating the product mixture and the extraction fluid after contacting for a heating time.

9. A method according to any one of the preceding claims, wherein after the separation step the organic phase is at least partially evaporated to recover the oleagineous compounds.

10. A method according to claim 9, wherein after the separation step the organic phase is fractionated at least partially to individually recover specific oleagineous compounds.

11. A method according to any one of the preceding claims, wherein the aqueous biomass suspension includes algal and/or bacterial biomass, sludge, manure, food, feedstock byproducts or mixtures thereof.

12. A method according to any one of the preceding claims, wherein the reaction temperature is 175 to 450 °C, the reaction pressure is 10 to 30 MPa and/or the reaction time is 2 to 60 min.

13. A method according to any one of the preceding claims, wherein the reactor is a continuous flow reactor.

14. Use of at least one extraction agent in a method of recovering oleagineous compounds from hydrothermally treated biomass that is provided as aqueous biomass suspension and processed at a reaction temperature and a reaction pressure for a reaction time in a reactor to produce a product mixture, **characterized in that** the at least one extraction agent is selected from the group consisting of monoaromatic hydrocarbons, monoaromatic alcohols, monoaromatic ethers and mixtures thereof and **in that** the at least extraction agent is used in an extraction fluid which is contacted with the product mixture in a separation step to obtain an organic phase that contains at least a part of the oleagineous compounds.

15. An use according to claim 14, wherein the at least one extraction agent is selected from the group consisting of toluene, benzyl alcohol, methoxybenzene, ethoxybenzene, m-xylene, o-xylene, p-xylene and mesitylene.
